# EUROPEAN PATENT SPECIFICATION

(11) **EP 2 280 734 B1**
(45) Date of publication and mention of the grant of the patent: **26.02.2014**
(21) Application number: 09734192.9
(22) Date of filing: 24.04.2009
(51) Int. Cl.: A61K 47/48, A61K 38/36, C12N 9/64, A61P 7/04

(54) **FACTOR IX CONJUGATES WITH EXTENDED HALF-LIVES**
FAKTOR-IX-KONJUGATE MIT VERLÄNGERTEN HALBWERTSZEITEN
CONJUGUÉS DE FACTEUR IX DOTÉS DE DEMI-VIES PROLONGÉES

(30) Priority: 24.04.2008 US 47544
(43) Date of publication of application: 09.02.2011
(73) Proprietor: Cantab Biopharmaceuticals Patents Limited, Valletta VLT 1436 (MT)
(72) Inventor: HENRY, William, County Bucks HP17 8LA (GB)
(74) Representative: Bassil, Nicholas Charles
(86) International application number: PCT/IB2009/005763
(87) International publication number: WO 2009/130602

(56) References cited:
- WO-A-2004/060965
- WO-A-2004/060966
- WO-A-2004/063250
- WO-A-2006/005058
- WO-A-2007/135182

## Description

### 1. FIELD OF INVENTION

The present invention relates to biocompatible polymer conjugates of Factor IX, compositions comprising Factor IX conjugates and uses of such conjugates for treating hemophilia. Pegylated Factor IX is for example disclosed in WO2007/135182.

### 2. SUMMARY OF THE INVENTION

The Applicant has determined that the manufacture of recombinant Factor IX (herein referred to as FIX) may be enhanced by conjugating FIX to one or more polyethylene glycol groups as defined in the claims so that FIX can be effectively separated from Factor IXa (herein referred to as FIXa). The enhanced manufacturing properties also include the ability to produce high purity FIX conjugates.

FIX conjugates may provide therapeutic benefits, for example, when compared to unconjugated FIX. Such therapeutic benefits include, but are not limited to, increased circulation half-life, reduced immunogenicity, higher activity, lower dosing requirements, and allowing for alternative routes of administration (e.g., subcutaneously).

As used herein, the terms "Factor IX conjugate" or "FIX conjugate" refers to Factor IX that has been modified to include a biocompatible polymer polyethylene glycol (PEG) containing moiety that results in an improved pharmacokinetic profile as compared to the unmodified Factor IX. The improvement in the pharmacokinetic profile may be observed as an improvement in one or more of the following parameters: potency, stability, area under the curve and circulating half-life.

In a specific embodiment, the high purity FIX conjugates described herein are substantially free of FIXa. Considered a contaminant of FIX compositions, even trace amounts of FIXa can result in dangerous thrombosis according to Gray et al. Thromb. Haemost. 1995 Apr; 73(4):675-9).

As used herein, the term "substantially free of FIXa," in the context of purifying FIX and FIX conjugates, refers to quantities of FIXa that are below 1u FIXa/1000 IU FIX. Small concentrations of FIXa can be measured according to the techniques described in Gray et al. Thromb. Haemost. 1995 Apr; 73(4):675-9. In some embodiments, the amount of FIXa contaminant may be less than 0.5 u FIXa/1000 IU FIX. In some embodiments, the amount of FIXa contaminant may be less than 0.25 u FIXa/1000 IU FIX. In some embodiments, the amount of FIXa may be less than 0.1 u FIXa/1000 IU FIX.

The conjugation of FIX with a polyethylene glycol enhances the separability of the FIX conjugate from FIXa, and thus enhances the utility of FIX in pharmaceutical compositions. Moreover, the polyethylene glycol may protect FIX from degradation and antibody response. The FIX conjugates may have a prolonged circulating half-life, which results in a dose-sparing effect and less frequent administration. In the invention, FIX is conjugated to one or more polyethylene glycol (PEG) moieties as defined in the claims.

Thus in a first aspect, the invention provides a Factor IX-polyethylene glycol (PEG) conjugate, wherein one or more polyethylene groups are bound to Factor IX through a PEG containing moiety which bridges two cysteine residues that form a disulfide bond in native Factor IX.

Therefore, the PEG containing linker bridges the disulfide bond. In a specific embodiment, a FIX-PEG conjugate may be according to the formula: 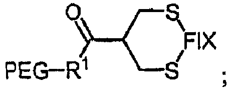
wherein R¹ represents a linking group which can be a direct bond, an alkylene group (preferably a C₁₋₁₀ alkylene group), or an optionally-substituted aryl or heteroaryl group; wherein the aryl groups include phenyl, benzoyl and naphthyl groups;
wherein suitable heteroaryl groups include pyridine, pyrrole, furan, pyran, imidazole, pyrazole, oxazole, pyridazine, pyrimidine and purine;
wherein linkage to the polymer may be by way of a hydrolytically labile bond, or by a non-labile bond.

Substituents which may be present on an optionally substituted aryl or heteroaryl group include for example one or more of the same or different substituents selected from - CN, -NO₂, -CO₂R, -COH, -CH₂OH, -COR, -OR, -OCOR, -OCO₂R, -SR, -SOR, -SO₂R,-NHCOR, -NRCOR, -NHCO₂R, -NR'CO₂R, -NO, -NHOH, -NR'OH, -C=N-NHCOR, -C=N-NR'COR, -N⁺R₃, -N⁺H₃, -N⁺HR₂, -N⁺H₂R, halogen, for example fluorine or chlorine, -C=CR, -C=CR₂ and ¹³C=CHR, in which each R or R' independently represents a hydrogen atom or an alkyl (preferably C₁₋₆) or an aryl (preferably phenyl) group. The presence of electron withdrawing substituents is especially preferred.

In another embodiment, PEG is conjugated to FIX according to the formula:

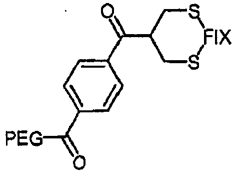

The PEG containing moiety may have a molecular weight of about 10 kDa. The FIX-PEG conjugate may have one PEG-containing moiety. Alternatively, the FIX-PEG conjugate may have two PEG-containing moieties.

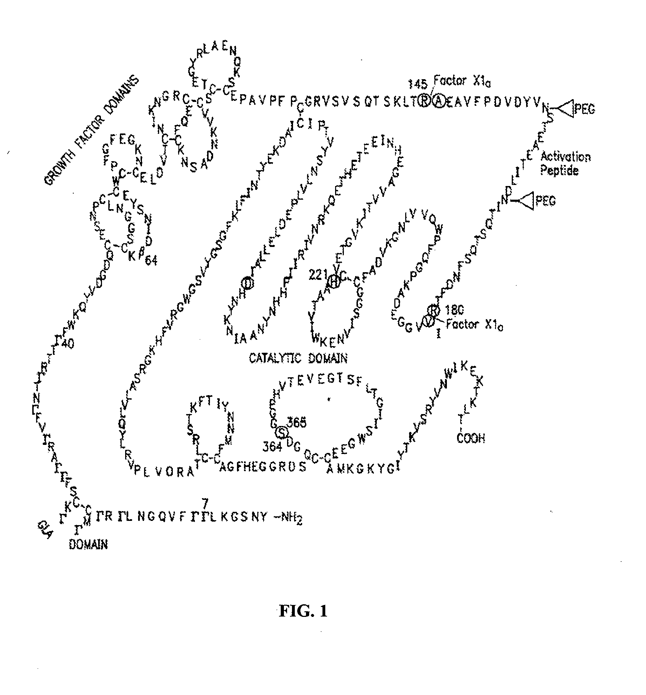

Conjugates of FIX may be manufactured such that they are substantially free of FIXa. Under certain conditions the rates at which FIX and FIXa react with a biocompatible polymer substrate may be different. Without being bound by theory, the difference in reaction rate may be due to the different size (FIX has a molecular weight that is 11kDa greater than FIXa) and/or different conformational structures of FIX and FIXa. The structure of FIX is shown in **Figure 1****,** which includes the activation domain that is cleaved upon FIX activation to FIXa. As a result, the residue side chain that is being conjugated may be more sterically accessible to the polymer containing reactant in FIX or FIXa, and the more accessible residue will react faster with the polymer containing reactant. This difference in reactivity ultimately allows for a kinetic separation of FIX and FIXa.

Recombinantly produced FIX may contain small quantities of FIXa as a contaminant. Sometimes these quantities can be extremely small, on the order of 1u FIXa/1000 IU FIX (Gray et al. Thromb. Haemost. 1995 Apr; 73(4): 675-9). Under some conditions the rate of formation of a FIX conjugate, such as FIX-PEG, may be faster than the rate of formation for FIXa-PEG. In this situation, a stoichiometric or sub-stoichiometric amount of the PEG reagent relative to FIX may be added to the reaction mixture. In this instance, all of the PEG reagent is consumed in the formation of the FIX-PEG conjugate, while FIXa remains unreacted. Properties of the FIX-PEG conjugate, such as molecular weight and polarity, allow it to be readily separated from FIXa using purification techniques known in the art, including but not limited to size exclusion and/or ion exchange chromatography may be used in the purification step.

Under some conditions the rate of formation of a FIXa conjugate, such as FIXa-PEG, is faster than the rate of formation for FIX-PEG. Under these conditions, stoichiometric excess of the PEG reagent relative to FIXa may be added to the reaction mixture. In this instance, it is important to ensure that all of the FIXa is consumed in the formation of FIXa-PEG, while most FIX, if not all FIX remains unreacted in its unconjugated state. The properties of the FIXa-PEG conjugate, such as molecular weight and polarity, allow it to be readily separated from unconjugated FIX using techniques known in the art. In one embodiment, the unconjugated FIX is purified by size exclusion or gel filtration chromatography. The resulting pure FIX can then be conjugated to PEG, which results in a pure FIX-PEG conjugate.

The FIX conjugates have one or more of the biological activities of unmodified FIX (*i.e.,* FIX without PEG attached). A FIX conjugate can be activated *in vivo* by Factor XIa or VIIa and tissue factor, to yield Factor IXa. The biological activity of the FIX conjugates may be determined using the assays described herein.

Compared to unmodified FIX, the FIX conjugates of the invention may show an improvement in one or more parameters of the pharmacokinetic profile, including area under the curve (AUC), Cₘₐₓ, clearance (CL), half-life, plasma residence time and bioavailability as compared to unmodified FIX.

The "area under the curve" or "AUC", as used herein in the context of administering a peptide drug to a patient, is defined as total area under the curve that describes the concentration of drug in systemic circulation in the patient as a function of time from zero to infinity.

As used herein the term "clearance" or "renal clearance" is defined as the volume of plasma that contains the amount of drug excreted per minute.

As used herein the term "half-life" or "t_{1/2}," in the context of administering a peptide drug to a patient, is defined as the time required for plasma concentration of a drug in a patient to be reduced by one half. There may be more than one half-life associated with the peptide drug depending on multiple clearance mechanisms, redistribution, and other mechanisms well known in the art. Usually, alpha and beta half-lives are defined such that the alpha phase is associated with redistribution, and the beta phase is associated with clearance. However, with protein drugs that are, for the most part, confined to the bloodstream, there can be at least two clearance half-lives. The precise impact of PEGylation on alpha phase and beta phase half-lives will vary depending upon the size and other parameters, as is well known in the art. Further explanation of "half-life" is found in Pharmaceutical Biotechnology (1997, DFA Crommelin and RD Sindelar, eds., Harwood Publishers, Amsterdam, pp 101 120).

As used herein the term "residence time," in the context of administering a peptide drug to a patient, is defined as the average time that drug stays in the body of the patient after dosing.

Compositions comprising FIX conjugates, which are substantially free of FIXa demonstrate improved safety. Furthermore, compositions comprising FIX conjugates, which are substantially free of FIXa are less likely to result in thrombosis. In a specific embodiment, the compositions comprising pure FIX conjugates are less likely to result in venous thrombosis. In another specific embodiment, the compositions comprising pure FIX conjugates are less likely to result in arterial thrombosis.

The FIX conjugates of the present invention are useful in treating hemophilia B. As used herein, the terms "treat", "treating" or "treatment of" mean that the severity of a subject's condition is reduced or at least partially improved or ameliorated and/or that some alleviation, mitigation or decrease in at least one clinical symptom is achieved and/or there is an inhibition or delay in the progression of the condition and/or delay in the progression of the onset of disease or illness. The terms "treat", "treating" or "treatment of" also means managing the disease state, e.g., hemophilia B.

The invention also provides a pharmaceutical composition comprising a FIX conjugate of the invention for use in treating patients suffering from hemophilia B.

As used herein, a "therapeutically effective" amount as used herein is an amount that provides some improvement or benefit to the subject. Alternatively stated, a "therapeutically effective" amount is an amount that provides some alleviation, mitigation, and/or decrease in at least one clinical symptom. Clinical symptoms associated with the disorder that can be treated by the methods of the invention are well-known to those skilled in the art. Further, those skilled in the art will appreciate that the therapeutic effects need not be complete or curative, as long as some benefit is provided to the subject.

In a specific embodiment, the pharmaceutical compositions comprising a FIX conjugate of the invention that is free of FIXa are for use in reducing the risk of hemarthrosis, haemorrhage, gastrointestinal bleeding and menorrhagia in mammals with haemophilia B.

As used herein, the "mean thrombus score," is defined as follows. Thrombi can be scored on a scale of 0-4. A score of 0 represents completely fluid blood; the presence of small fibrin dots are scored a 1, several larger pieces of clot are scored a 2; a clot that is partially occluded is scored as a 3, and a score of 4 represents a solid cast of the segment. The mean thrombus score of the composition may be less than 1, less than 0.5 or 0 after three independent experiments. The mean thrombus score should be determined from three or more independent experiments following the protocol from Wessler et al, J. Appl. Physiol. 14:943-946 (1959).

In another specific embodiment, the improved properties of FIX conjugates provide a method for the prophylactic treatment of hemophilia B. In another specific embodiment, the compositions comprising a FIX conjugate may be administered to patients suffering from hemophilia B, who are undergoing a surgical procedure or recovering from a surgical procedure.

The administration of FIX conjugates to a patient for the treatment of hemophilia B may be combined with other therapeutics, such as, tranexamic acid, aminocaproic acid, Factor II (prothrombin) and/or Factor X (Stuart Prower Factor).

The FIX conjugates, described herein, may be formulated with a pharmaceutically acceptable carrier. As used herein, the term "pharmaceutically acceptable" when used in reference to the formulations of the present invention denotes that a formulation does not result in an unacceptable level of irritation in the subject to whom the formulation is administered by any known administration regimen. Examples of irritation include hypersensitivity reactions to FIX.

Due to the increased half-life of FIX conjugates, the pharmaceutical compositions may contain a lower dose of FIX than typically administered to effectively treat hemophilia. The pharmaceutical formulations of the invention may be formulated for parenteral administration, including, but not limited to, intradermal, subcutaneous, and intramuscular injections, and intravenous or intraosseous infusions. The pharmaceutical formulations of the present invention can take the form of solutions, suspensions, emulsions that include a FIX-PEG conjugate, and a pharmaceutically acceptable diluent, adjuvant or carrier, depending on the route of administration.

The pharmaceutical compositions of the invention are formulated to deliver a therapeutic dose of a FIX conjugate. The dosage of the FIX conjugate may be expressed in international units (IU). As used herein, the term "international unit" or "IU" refers to the potency assignment that is referenced to the World Health Organization International Standard. According to this assignment, one IU of Factor IX activity per kg of body weight is approximately equal to the Factor IX activity in 1 mL of pooled normal human plasma, and increases the plasma concentration of Factor IX by 1%.

The dose of the FIX conjugates contained in pharmaceutical formulation can range from 1 IU to 10,000 IU. In certain embodiments, the dose of the FIX conjugate can range from 100 IU to 5000 IU, or 200 IU to 2500 IU. In certain embodiments, the dose of the FIX conjugate can be about 250 IU, about 500 IU, about 1000 IU or about 2000 IU.

The FIX conjugate is administered in an amount sufficient to treat hemophilia B. As used herein, a "sufficient amount" or an "amount sufficient to" achieve a particular result refers to an amount of a FIX conjugate that is effective to produce a desired effect, which is optionally a therapeutic effect (*i.e*., by administration of a therapeutically effective amount). For example, a "sufficient amount" or "an amount sufficient to" can be an amount that is effective to reduce the risk of hemarthrosis, hemorrhage, gastrointestinal bleeding and menorrhagia.

In certain embodiments, the dosage of a FIX conjugate is a sufficient such that the concentration of circulating Factor IX activity is 1 IU/kg to 150 IU/kg. In another embodiment, the composition is administered at a dose such that the concentration of circulating Factor IX activity is 10 IU/kg to 120 IU/kg. In another embodiment, the composition is administered at a dose such that the concentration of circulating Factor IX activity is 20 IU/kg to 100 IU/kg.

The conjugates of the invention can be used in the same manner as unmodified FIX. However, because of the improved properties of FIX conjugates, the pharmaceutical formulations of the invention can be administered less frequently than unconjugated FIX. For example, the FIX conjugates may be administered once weekly instead of the once daily for unmodified recombinant FIX. The present invention also encompasses dosing regimens wherein the FIX derivatives may be administered twice a day, once a day, once every two, three or four days, or once a week to effectively treat hemophilia B. Decreased frequency of administration is expected to result in improved patient compliance leading to improved treatment outcomes, as well as improved patient quality of life.

### 3. BRIEF DESCRIPTION OF THE DRAWINGS

**FIG. 1** is a schematic representation of Factor IX showing the amino acid sequence, structure and various domains.
**FIG. 2** illustrates the circulation half-life of unconjugated FIX-PEG in mouse serum.
**FIG. 3** illustrates the circulation half-life of 10kDa FIX-PEG in mouse serum.
**FIG. 4** illustrates the circulation half-life of 20kDa FIX-PEG in mouse serum.

### 4. DETAILED DESCRIPTION OF THE INVENTION

The present invention describes FIX conjugated to one or more polyethylene glycol polymers in order to provide a FIX conjugate that is substantially free of FIXa. The FIX conjugates described herein also have improved biological and pharmacokinetic properties including, but not limited to increased circulating half-life or plasma residence time as compared to unmodified FIX. Furthermore, the conjugates described herein are less susceptible to antibody response. The present invention is also related to methods of preparing such conjugates. The FIX conjugate is a FIX-PEG conjugate. The present invention further relates to methods of using FIX conjugates for treatment of hemophilia B.

In the FIX-PEG conjugate of the invention, one or more PEG groups are conjugated to FIX through a PEG containing moiety which bridges two cysteine residues that form a disulfide bond in native FIX. These conjugates may be produced via reductive cleavage of a disulfide bond, followed by a reaction in which the PEG moiety becomes bound to both thio groups. The resulting FIX conjugate contains a PEG moiety that bridges two sulfurs that had formed a disulfide bond.

The compositions of the invention comprise FIX-PEG conjugates that are substantially free of FIXa. The compositions of the invention have a reduced thrombus score, compared to compositions FIX that contains trace contaminants of FIXa, thereby reducing the risk of thrombolytic events including, but are not limited to, venous and/or arterial thrombosis.

Also described herein is a method of purifying FIX from FIXa by taking advantage of the differing rates of formation of FIX conjugates versus FIXa conjugates in conjugation reactions with biocompatible polymer moieties. This results in the kinetic separation of FIX and FIXa. In one embodiment the biocompatible polymer is PEG.

The FIX conjugates of the present invention have an improved pharmacokinetic profile as compared to unmodified FIX.

Another aspect of the invention is a pharmaceutical composition comprising a FIX conjugate and a pharmaceutically acceptable diluent, adjuvant or carrier for use in treating hemophilia B. As used herein, when referring to the administration of FIX conjugates of the invention, the term a "patient in need thereof," refers to a patient who has been diagnosed with hemophilia B and/or is deficient in Factor IX.

### 4.1 Recombinant Factor IX

Recombinant Factor IX produced by any methods known in the art are contemplated for derivatizing and conjugation in accordance with the present invention. Preferred FIX can be produced using methods set forth in one or more of the following patents and publications: U.S. Patent Nos. 5,268,275 , 5,171,569 , 5,888,809 , 6,531,298; International Application Publication Nos. WO 2005/030039, WO 2006/101474, WO 2006/089613; U.S. Application Publication Nos. 2003/0220247, 2005/0271644, 2006/0121574, and 2006/0194284.

In some embodiments, the FIX has been lyophilized or freeze-dried. In preferred embodiments, the FIX has not been lyophilized or freeze-dried. In some embodiments, the FIX has been exposed to cryoprotectants, e.g., sucrose or trehalose. In preferred embodiments, the FIX has not been exposed to cryoprotectants, e.g., sucrose or trehalose.

### 4.2 Factor IX Conjugates

Compared to unmodified FIX, the FIX conjugates of the invention may show an improvement in one or more parameters of the pharmacokinetic profile, including AUC, Cₘₐₓ, clearance (CL), half-life, plasma residence time and bioavailability as compared to unmodified FIX. The FIX conjugates may have increased clinical activity *in vivo* as compared to unmodified FIX. The conjugates of the invention may have improved potency and stability.

The FIX conjugates, described herein, can be purified and isolated from FIXa via chromatographic methods known in the art. The methods include, but are not limited to, ion exchange chromatography and size exclusion chromatography. The FIX conjugates of the invention have one or more of the biological activities of unmodified FIX. Conjugates of FIX can be activated *in vivo* by Factor XIa or VIIa and tissue factor, to yield Factor IXa. The biological activity of FIX conjugates may be determined using the assays described herein.

The FIX to be modified in accordance with the invention may be obtained and isolated from natural sources, such as human plasma. The FIX to be modified in accordance with the invention may be expressed recombinantly.

In one embodiment, the FIX component of the conjugate has the sequence identified as human FIX identified in Figure 1. Alternatively, the sequence of FIX may be modified or derivatized to include one or more changes in the amino acid sequence, including, but not limited to insertions, deletions or substitutions.

A FIX-PEG conjugate may lead to increased circulating half life and plasma residence time, decreased clearance, and increased clinical activity *in vivo.* FIX is modified by covalently binding a polyethylene glycol polymer through two cysteine amino acid residues as defined in the claims. Polyethylene glycol polymer units can be linear or branched.

The FIX-PEG conjugate may be delivered intravenously or subcutaneously via injection. The FIX-PEG conjugate may also be formulated for oral administration in a tablet, capsule, solution or suspension.

The PEG containing moiety bridges two cysteine thiol groups that form a disulphide bond in native FIX.

There are several different types of polyethylene glycol polymers that will form conjugates with FIX. There are linear PEG polymers that contain a single polyethylene glycol chain, and there are branched or multi-arm PEG polymers. Branched polyethylene glycol contains 2 or more separate linear PEG chains bound together through a unifying group. For example, two PEG polymers may be bound together by a lysine residue. One linear PEG chain is bound to the α-amino group, while the other PEG chain is bound to the ε-amino group. The remaining carboxyl group of the lysine core is left available for covalent attachment to a protein. Both linear and branched polyethylene glycol polymers are commercially available in a range of molecular weights.

In one aspect of the invention, a FIX-PEG conjugate contains one or more linear polyethylene glycol polymers bound to FIX, in which each PEG has a molecular weight between about 2kDa to about 100kDa. In another aspect of the invention, a FIX-PEG conjugate contains one or more linear polyethylene glycol polymers bound to FIX, wherein each linear PEG has a molecular weight between about 5kDa to about 40kDa. In certain embodiments, each linear PEG has a molecular weight between about 5kDa to about 20 kDa. In certain embodiments, each linear PEG has a molecular weight that is about 10 kDa. In certain embodiments, each linear PEG has a molecular weight that is less than about 10 kDa. In particular embodiments, there the FIX-PEG conjugate contains more than one linear PEG polymers bound to FIX, for example two, three, or four linear PEG polymers bound to FIX. In a some embodiments, the FIX-PEG conjugates contains two linear PEG polymers, where each linear PEG has a molecular weight of about 10 kDa.

A FIX-PEG conjugate of this invention may contain one or more branched PEG polymers bound to FIX, wherein each branched PEG has a molecular weight between about 2kDa to about 100kDa. In another aspect of the invention, a FIX-PEG conjugate contains one or more branched polyethylene glycol polymers bound to FIX, wherein each branched FIX has a molecular weight between about 5kDa to about 40kDa. In certain embodiments, each branched PEG has a molecular weight between about 5kDa to about 20 kDa. In certain embodiments, each branched PEG has a molecular weight that is about 10 kDa. In certain embodiments, each branched PEG has a molecular weight that is less than about 10 kDa. In particular embodiments, there the FIX-PEG conjugate contains more than one branched PEG polymers bound to FIX, for example two, three, or four branched PEG polymers bound to FIX. In a some embodiments, the FIX-PEG conjugates contains two branched PEG polymers, where each branched PEG has a molecular weight of about 10 kDa.

The FIX-PEG conjugates may be purified by chromatographic methods known in the art, including, but not limited to ion exchange chromatography and size exclusion chromatography.

### 4.3 Methods of Producing Factor IX Derivatives

### 4.3.1. Techniques for the PEGylation of Cysteine Residues

A number of methods exist in the art for forming polyethylene glycol conjugated, or PEGylated cysteine residues. The advantage of these techniques are that they are selective for cysteine, which means that other side chains remain unaffected by these methods. In **scheme 1a,** the activated disulphide, PEG ortho-pyridyl-disulphide, reacts with thiols to form the more stable symmetric disulphide. In **scheme 1b,** a cysteine residue reacts with PEG-maleamide, via a thiol addition to the activated double bond in a Michael addition reaction. In **scheme 1c** a conjugate attack by the thiol on the activated terminal vinyl group of PEG-vinylsulphone, yields the PEGylated cysteine residue. In **scheme 1d** the cysteine thiol displaces the iodide via a nucleophilic attack to yield the PEG conjugated cysteine resudue.

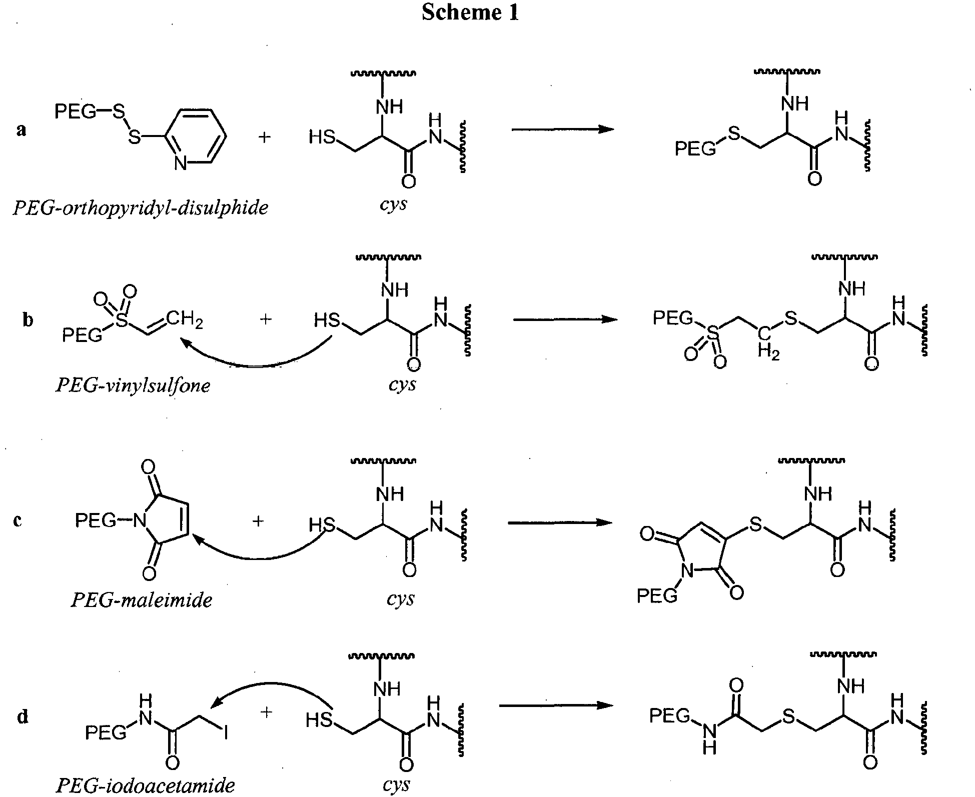

Two cysteine groups that together form a disulfide bond may also be PEGylated selectively by using the technique shown in **scheme 2.** The native disulfide bond is first reduced. One of the resulting thiols from this bond can nucleophilicly attack an electrophilic group, such as a 1,4-addition to an enone. This is followed by the departure of a leaving group, such as, e.g. a sulfone. The subsequent elimination to a second enone, followed by 1,4-addition by the remaining thiol leads to the bridged disulfide with a PEG group attached.

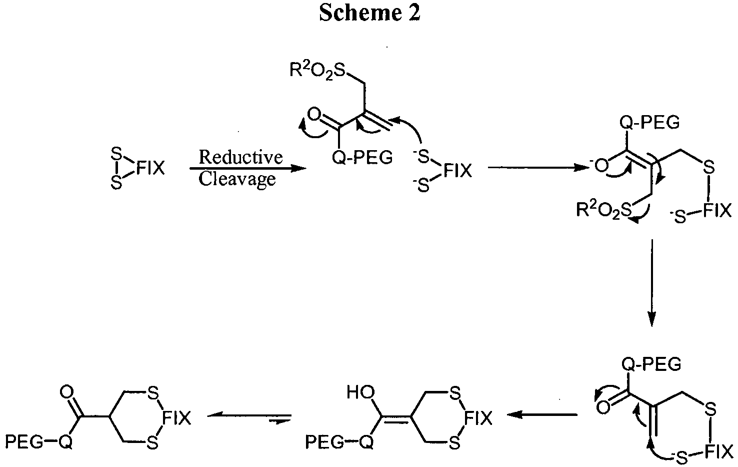

The technique shown in **scheme 2** can also be used to prepare a FIX conjugate with two or more independent PEG moieties conjugates thereto. For example, a first native disulfide bond is reduced under controlled conditions well known in the art. A first PEG moiety is then attached thereto in accordance with **scheme 2.** Subsequently, a second disulfide bond is reduced, for example, under more complete reduction conditions well known in the art. A second PEG moiety is then attached thereto, again in accordance with **scheme 2.** This multi-step procedure can be repeated as necessary.

### 4.3.2. Methods of Producing Highly Purified FIX

The conjugation of FIX to biocompatible polymers provides a method with which to produce highly purified FIX conjugates. In particular, methods of forming FIX conjugates in the presence of FIXa, while not forming FIXa conjugates are especially advantageous. Conjugates of FIX may be readily separated from FIXa by chromatographic methods such as ion exchange or size exclusion chromatography.

In one embodiment, the rate of formation of FIX-PEG occurs at faster rate than FIXa-PEG. The reaction for the selective conjugation of FIX is shown in scheme 3 below.

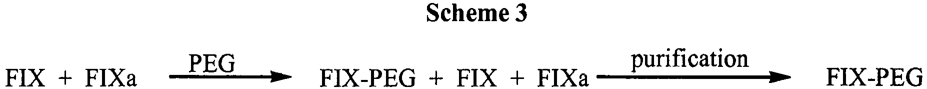

To a reaction mixture of FIX, in the presence of a trace amount of FIXa, is added a stoichiometric amount of a PEG reagent. In one embodiment, the PEG-reagent / FIX ratio in the reaction mixture is from about 0.1 to about 1.5. In another embodiment, the PEG-reagent / FIX ratio is from about 0.1 to about 1.0. In another embodiment, the PEG-reagent / FIX ratio is from about 1.0 to about 1.5. In another embodiment, the PEG-reagent / FIX ratio is from about 0.1 to about 0.5. In another embodiment, the PEG-reagent / FIX ratio is from about 0.5 to about 0.7. In another embodiment, the PEG-reagent / FIX ratio is from about 0.7 to about 1.0.

In one embodiment, the PEG reagent may be added to the reaction mixture in one charge. In another embodiment, the PEG-reagent is added slowly over time. The PEG-reagent may be added over a period of up to about 1, 2, 6, 12, 18 or 24 hours. The progress of the reaction may be monitored by methods including, but not limited to mass spectroscopy, fast protein liquid chromatography (FPLC) or dodecyl sulfate polyacrylamide gel electrophoresis (SDS-PAGE). It is important to ensure that FIXa is not being converted to a FIXa-PEG conjugate. In one embodiment, FIX is nearly quantitatively converted to FIX-PEG. In another embodiment, FIX is converted to FIX-PEG at approximately 80% to 90% conversion, 70% to 80% conversion, 60% to 70% conversion, 50% to 60% conversion, 40% to 50% conversion, 30% to 40% conversion, 30% to 40% conversion, 20% to 30% conversion or 10% to 20% conversion. Unreacted FIX may be recycled, and undergo additional conjugation reactions.

A FIX-PEG conjugate may be readily separated from the reaction mixture via ion affinity or size exclusion chromatography. After the separation step, it is important to ensure that the resulting FIX-PEG conjugate is substantially free of FIXa. This can be confirmed by the methods described in Gray et al. Thromb. Haemost. 1995 Apr; 73(4):675-9, or the methods in Section 5.3 below.

In another embodiment the rate of formation of FIXa-PEG conjugate occurs faster than for FIX-PEG. Unreacted FIX is then separated from the FIXa-PEG conjugate by chromatographic methods described herein. In order to produce pure FIX-PEG, the remaining purified FIX may undergo a further PEGylation reaction. Scheme 4, shows how purified FIX-PEG may be synthesized under this circumstance.

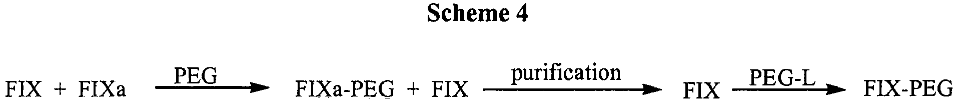

Under these conditions, a stoichiometric excess of the PEG reagent relative to FIXa may be added to the reaction mixture. In one embodiment, the PEG-reagent / FIXa ratio in the reaction mixture is from about 100 to about 1000. In another embodiment, the PEG-reagent / FIX ratio is from about 10 to about 100. In another embodiment, the PEG-reagent / FIX ratio is from about 1.1 to about 10.

It is important to ensure that all of the FIXa is consumed in the formation of FIXa-PEG. The properties of the FIXa-PEG conjugate, such as molecular weight and polarity, allow it to be readily separated from unconjugated FIX using techniques known in the art. In one embodiment, the unconjugated FIX is purified by size exclusion or gel filtration chromatography.

### 4.4 Methods of Assaying Biological Activity

The FIX conjugates of the invention have one or more of the biological activities of unmodified FIX. Methods for determining the activity of a FIX conjugate prepared according to the methods of the present invention can be carried out using methods well known in the art, such as a one stage activated partial thromboplastin time assay as described in, for example, Biggs (1972, Human Blood Coagulation Haemostasis and Thrombosis (Ed. 1), Oxford, Blackwell, Scientific, pg. 614). Briefly, to assay the biological activity of a FIX conjugate developed according to the methods of the present invention, the assay can be performed with equal volumes of activated partial thromboplastin reagent, FIX deficient plasma isolated from a patient with hemophilia B using sterile phlebotomy techniques well known in the art, and normal pooled plasma as standard, or the sample. In this assay, one unit of activity is defined as that amount of activity present in one milliliter of normal pooled plasma. Further, an assay for biological activity based on the ability of FIX to reduce the clotting time of plasma from FIX-deficient patients to normal can be performed as described in, for example, Proctor and Rapaport (1961, Amer. J. Clin. Path. 36: 212). The biological activity of FIX conjugates to ensure that there is no FIXa contamination may be determined using the assays described in section 5.3 as well.

### 4.4.1. Pharmaceutical Compositions

The present invention relates to pharmaceutical compositions containing a FIX conjugate as the active ingredient. The FIX conjugate may be formulated with a pharmaceutically acceptable carrier. Due to the increased half-life of the FIX conjugate, the pharmaceutical compositions may contain a lower dose of FIX than typically administered to effectively treat hemophilia B. The pharmaceutical formulations of the invention may be formulated for parenteral administration, including, but not limited to, intradermal, subcutaneous, and intramuscular injections, and intravenous or intraosseous infusions. The pharmaceutical formulations of the present invention can take the form of solutions, suspensions or emulsions that include a FIX conjugate, such as FIX chemically modified with polyethylene glycol, and a pharmaceutically acceptable diluent, adjuvant or carrier, depending on the route of administration.

The pharmaceutical compositions of the invention are formulated to deliver a therapeutic dose of a FIX conjugate. The dosage of the FIX conjugate may be expressed in international units (IU). The dose of the FIX conjugates contained in pharmaceutical formulation can range from 1 IU to 10,000 IU. In certain embodiments, the dose of the FIX conjugate can range from 100 IU to 5000 IU, or 200 IU to 2500 IU. In certain embodiments, the dose of the FIX conjugate can be about 250 IU, about 500 IU, about 1000 IU or about 2000 IU.

In some embodiments, the pharmaceutical compositions of the invention display an advantageous immunogenicity profile, for example, compared to an analogous pharmaceutical composition of unconjugated FIX. In certain embodiments, the pharmaceutical compositions of the invention are substantially less immunogenic than analogous pharmaceutical composition of unconjugated FIX, or substantially non- immunogenic. In related embodiments, such pharmaceutical compositions with advantageous immunogenicity profiles are capable of being administered to a patient subcutaneously, for example, in a method of treating hemophilia B.

### 4.5 Treating Hemophilia B

The FIX conjugates described herein may be used in treating hemophilia B. The pharmaceutical compositions of the invention are formulated to deliver a therapeutic dose of a FIX conjugate. In certain embodiments, the dosage of a FIX conjugate is sufficient such that the concentration of circulating FIX activity in a subject is 1 IU/dL to 150 IU/dL. In another embodiment, the composition is administered at a dose such that the concentration of circulating FIX activity in a subject is 10 IU/dL to 120 IU/dL. In another embodiment, the composition is administered at a dose such that the concentration of circulating FIX activity in a subject is 20 IU/dL to 100 IU/dL.

In some embodiments, the activity of the FIX conjugate is about 100% of that of an analogous unconjugated FIX. In some embodiments, the activity of the FIX conjugate is greater than about 80%, 60%, or 40% of that of an analogous unconjugated FIX. In preferred embodiments, the activity of the FIX conjugate is greater than about 20% of that of an analogous unconjugated FIX. While not preferred, the activity can be lower than 20%, such as 10%, 5% or even 1% and may still be advantageously used compared to non conjugated FIX.

For patients receiving the FIX conjugate, their FIX activity may need to be monitored by a medical professional. The FIX conjugates described herein may also be administered to patients suffering from hemophilia B who are undergoing or recovering from a surgical procedure.

Furthermore, the FIX conjugates described herein are free of contamination from FIXa, which may lead to adverse thrombolytic incidents. Therefore, the FIX conjugates and methods of manufacturing FIX conjugates described herein yield pure FIX conjugates that are less likely to cause adverse thrombosis as determined by the mean thrombus score. In one embodiment, the mean thrombus score of the FIX conjugate composition is less than 1 after three or more independent measurements. In another embodiment, the mean thrombus score is less than 0.5, after three or more independent measurements. In another embodiment, the mean thrombus score, after three or more independent measurements, is less than 0.3. In another embodiment, the mean thrombus score, after three or more independent measurements, is less than 0.1. In yet another embodiment, the mean thrombus score is 0, after three or more independent measurements.

In some embodiments, the administered dose of the FIX conjugate is approximately the same as that necessary for an analogous unconjugated FIX. In some embodiments, the administered dose of the FIX conjugate is about two times, about three times, about our times, or about five times that necessary for an analogous unconjugated FIX.

Also described herein is a FIX conjugate and an additional therapeutic agent for use in treating hemophilia B. The additional therapeutic agent may be one or more of tranexamic acid, aminocaproic acid, Factor II (prothrombin) and/or Factor X (Stuart Prower Factor).

The FIX conjugate and the additional therapeutic agent can be administered sequentially or simultaneously. If administered sequentially, the order of administration is flexible. For instance, the FIX conjugate can be administered prior to administration of the additional therapeutic agent. Alternatively, administration of the additional therapeutic agent can precede administration of the FIX conjugate.

Whether they are administered as separate compositions or in one composition, each composition is preferably pharmaceutically suitable for administration. Moreover, the FIX conjugate and the therapeutic agent, if administered separately, can be administered by the same or different modes of administration.

In certain embodiments, the FIX conjugates of the invention exhibit substantially the same pharmaceutical effects as an analogous unconjugated FIX. For example, in some embodiments, administration of the FIX conjugates of the invention lead to normal fibrin-induced clotting, indicating coagulation.

### 4.5.1. Dosing Regimens

Dosing regimens include administration of the conjugates of the invention twice daily, once a day, every other day, twice weekly, once weekly, once every two weeks or once a month to a patent suffering from hemophilia B.

A medical professional may monitor the FIX activity in a subject receiving FIX conjugate, and vary the dosing regimen accordingly. In one embodiment, the FIX activity in a subject is determined immediately after administration of a FIX conjugate. In another embodiment, the FIX activity in a subject is determined 0.5 hr., 1 hr., 6 hrs., 12 hrs., or 24 hrs. after administration of a FIX conjugate. In another embodiment, the FIX activity in a subject is determined 2 days, 4 days, 1 week, 2 weeks or 3 weeks after administration of a FIX conjugate.

### 5. EXAMPLES

### 5.1 Syntheses of FIX Conjugates

The FIX conjugates of the invention can be readily synthesized using synthetic methods known in the art. The following synthetic examples demonstrate the syntheses of FIX-PEG conjugates.

### 5.1.1. Example 1: PEGylation of the FIX via Disulfide Bond Bridging

To Factor IX is added aqueous urea solution 2-mercaptoethanol. The pH of the resulting solution is adjusted to pH 8.5 using a 10% aqueous solution of methylamine. The reaction solution is then bubbled with nitrogen for approximately 30 min. Still purging with nitrogen the tube is heated at 37°C. The reaction mixture is then cooled in an ice-salt water bath and 10 mL of an argon purged chilled solution of 1N HCl:absolute ethanol is added to the reaction solution. A precipitation occurs and the precipitate is isolated by centrifugation and then washed three times with further portions of the HCl:absolute ethanol mixture and twice with nitrogen purged chilled diethyl ether. After each washing the precipitate is isolated by centrifugation. The washed precipitate is then dissolved in nitrogen purged deionized water and freeze-dried to afford a dry solid. Partial reduction of FIX may be confirmed and quantitated using Ellman's Test, which gives the number free thiols per protein molecule.

In an eppendorf, the partially reduced FIX is dissolved in argon purged pH 8 ammonia solution. In a separate eppendorf, the polymer conjugating reagent, α-methoxy-ω-4-[2,2-bis[(p-tolylsulfonyl)-methyl]acetyl]benzamide derived from poly(ethylene)glycol is also dissolved in ammonia solution and the resulting solution is added to the Factor IX solution. The PEG eppendorf is washed with fresh ammonia solution and this is also added to the main reaction eppendorf. The reaction eppendorf is then closed under argon and heated at 37°C for approximately 24 h and then allowed to cool to room temperature. The cooled reaction solution is then analyzed by sodium dodecyl sulfate-polyacrylamide gel electrophoresis (SDS-PAGE).

### 5.1.2. Example 2: Selective formation and Purification of FIX-PEG in the presence of FIXa Contaminant

To a reaction mixture containing FIX that is contaminated with FIXa is added aqueous urea solution 2-mercaptoethanol. The pH of the resulting solution is adjusted to pH 8.5 using a 10% aqueous solution of methylamine. The reaction solution is then bubbled with nitrogen for approximately 30 min. Still purging with nitrogen the tube is heated at 37°C. The reaction mixture is then cooled in an ice-salt water bath and 10 mL of an argon purged chilled solution of 1N HCl:absolute ethanol is added to the reaction solution. A precipitation occurs and the precipitate is isolated by centrifugation and then washed three times with further portions of the HCl:absolute ethanol mixture and twice with nitrogen purged chilled diethyl ether. After each washing the precipitate is isolated by centrifugation. The washed precipitate is then dissolved in nitrogen purged deionized water and freeze-dried to afford a dry solid. Partial reduction of FIX may be confirmed and quantitated using Ellman's Test, which gives the number free thiols per protein molecule.

In an eppendorf, the partially reduced FIX is dissolved in argon purged pH 8 ammonia solution. In a separate eppendorf, less the one equivalent of the polymer conjugating reagent, α-methoxy-ω-4-[2,2-bis[(p-tolylsulfonyl)-methyl]acetyl]benzamide derived from poly(ethylene)glycol is also dissolved in ammonia solution and the resulting solution is added to the FIX solution. The PEG eppendorf is washed with fresh ammonia solution and this is also added to the main reaction eppendorf. The reaction eppendorf is then closed under argon and heated at 37°C. for approximately 24 h and then allowed to cool to room temperature. The cooled reaction solution is then analyzed by sodium dodecyl sulfate-polyacrylamide gel electrophoresis (SDS-PAGE). The FIX-PEG is then purified from the from the reaction mixture via size exclusion chromatography.

The isolated product may be further analyzed by the methods provided by Gray et al. Thromb. Haemost. 1995 Apr; 73(4):675-9 or section 5.3 below to determine the level of FIXa contaminant.

### 5.2 Production of Recombinant Factor IX

### 5.2.1. Example 3: Primary Transfection of CHO Cells With Factor IX Gene

A wild-type Factor IX gene is transfected into CHO cells by limit dilution into 96-well plates. The Factor IX gene is under the control of the CHEF-1 promoter. Cells are allowed to grow in 5% serum for 14 days. The cell culture medium is harvested and the total amount of Factor IX antigen in µg per mL is quantified by a Factor IX ELISA method. More than 150 clones are evaluated and the total amount of Factor IX produced per clone is determined.

CHO cells transfected with the Factor IX gene produce Factor IX antigen which is detected by Factor IX ELISA.

### 5.2.2. Example 4: Supertransfection of Factor IX-Producing CHO Cells With VKGC and VKOR Genes

In order to increase the percentage of active Factor IX produced in Factor XI-transfected CHO cells, the primary transfectants are pooled, expanded in tissue culture and supertransfected with vectors containing cDNA for enzymes generally thought to be important for the efficient Vitamin K-dependent gamma-carboxylation of Factor IX. Factor IX producing clones are pooled in a shake flask and supertransfected with cDNAs for both Vitamin K-dependent gamma-carboxylase (VKGC) and Vitamin K-dependent epoxide reductase (VKOR). Individually supertransfected cells are grown by limit dilution in 96-well plates in 5% serum for 14 days. The total amount of Factor IX antigen produced per mL is measured by Factor IX ELISA. The amount of active Factor IX is measured by an APTT clotting assay using Factor IX-deficient plasma as substrate and plasma-derived Factor IX as standard.

### 5.3 Bioassays of FIX Conjugates

### 5.3.1. Example 5: Assay for FIX-PEG and FIXa

The purified FIX-PEG composition is assayed for the determination of FIX-PEG activity as well as any contaminant FIXa or FIXa-PEG activities. The assays are conducted according to the protocols described in Smith, K. J. et al., Blood, 72, 1269-1277 (1988), for FIX and Varadi, K. et al., Thromb. Haemos., 35, 576-585 (1976), for FIXa contamination.

### 5.3.2. Example 6: FIX-PEG Composition in vivo Thrombogenicity Assay

This example is used to determine whether a FIX conjugate, purified in accord with the process of Example 2, causes unwanted coagulation due to FIXa contamination as measured by the in vivo Wessler Rabbit Stasis Assay for thrombogenicity.

FIX preparations are injected *in vivo* into isolated, ligated sections of rabbit jugular veins according to the procedure of Wessler et al., J. Appl. Physiol. 14:943-946 (1959) to assess the formation of stasis thrombi.

Scoring is accomplished following the system of Wessler, *et al.* according to the size of the clot wherein a ⁺4clot represents the largest size of clot which can normally be generated with thrombogenic materials in the size and type of vessel selected and ⁺1 is the smallest such clot which can be visibly detected. The experiment is repeated three times, and the arithmetic mean is determined.

### 5.3.3. Example 7: Detection of Trace FIXa and FIXa-PEG in Purified FIX-PEG compositions

Reference FIXa (25µL) diluted with TBS/1% human albumin or 25µL of test FIX concentrate dilutions in TBS/1% human albumin are placed into 96 well microliter plates at 37°C. An assay reagent containing equal volumes human FX, bovine brain phospholipid, and recombinant desulphatohirudin is warmed for 5 minutes at 37°C. Equal volumes of warmed recombinant FVIII and CaCl₂ are then added to the assay reagent. Immediately after mixing 125 µL of this reagent is added to wells containing FIX or FIXa samples. After 20 min FXa generation at 37°C, 50µL is transferred into 100µL of S-2765 (Quadratech Epsom UK). After 2 minutes of incubation the reaction was stopped by the addition of 50µL of 50% acetic acid and an absorbance wavelength is recorded. The level of absorbance for the test substrate is then carried out at this wavelength to determine the quantity of FIXa in the test mixture.

### 5.3.4. Example 8: Comparison of the Circulation Half-Life of 10kDa FIX-PEG and 20kDa FIX-PEG with Unconjugated FIX

10kDa FIX-PEG and 20 kDa FIX-PEG (where the PEG moiety in each is linear) were prepared using the procedures set forth in Examples 1 and 2.

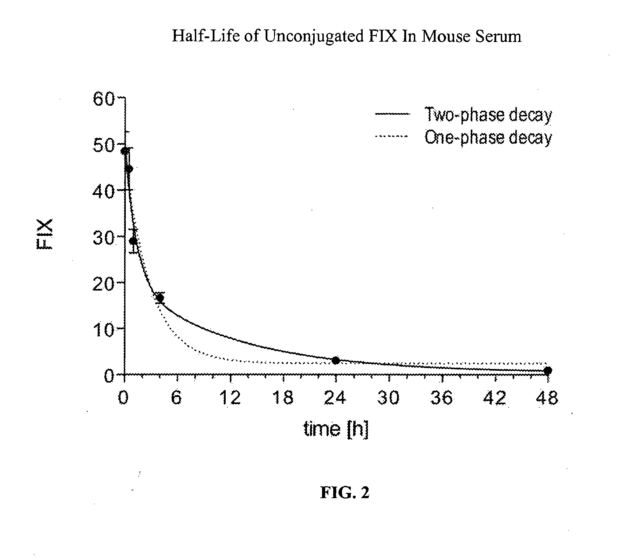

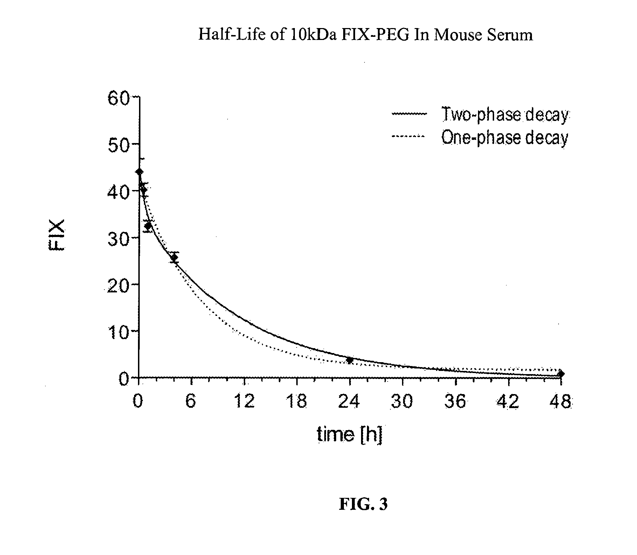

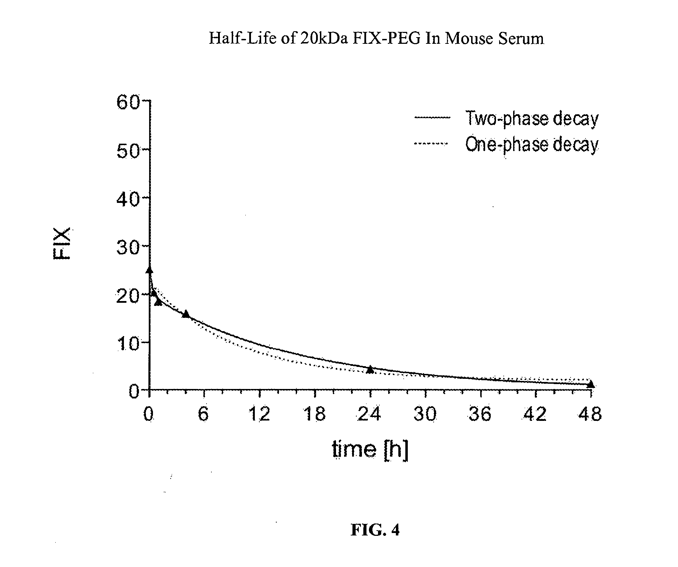

Unconjugated FIX, 10kDa FIX-PEG, and 20kDa FIX-PEG were administered to FIX-deficient mice, and the plasma concentration in each were measured both by ELISA and chromogenically from 15 minutes up to 48 hours after administration. The average plasma concentrations are shown in Figures 2, 3, and 4, respectively. When measured over the time period from about 15 minutes to about 4 hours after administration, the half-lives of 10kDa and 20kDa FIX-PEG were about 2-2.5 times longer than the half-life of unconjugated FIX. When measured over the time period from about 15 minutes to about 48 hours after administration, the half-life of 20kDa PEG-FIX was approximately 11 times greater than that of unconjugated FIX, and the half-life of 10kDa PEG-FIX was approximately 8 times greater than that of unconjugated FIX.

### 5.3.5. Example 9: Activity of 10kDa FIX-PEG and 20kDa FIX-PEG in FIX-Deficient Mice

The activity of 10kDa FIX-PEG and 20kDa FIX-PEG was examined by performing a bleed-out study with FIX-deficient mice. The mice were administered the equivalent of 45 µg/mL dose of unconjugated FIX. Mice tails were clipped, and 10kDa FIX-PEG and 20kDa FIX-PEG was administered to 12 and 16 mice, respectively. Death was prevented for 11 of the 12 mice in the 10kDa FIX-PEG group, and death was prevented for 12 of the 16 mice in the 20kDa FIX-PEG group. Cessation of bleeding was observed by visual inspection to be due to the formation of a fibrin clot, as opposed to a platelet-only cessation. These results indicate that both 10kDa FIX-PEG and 20kDa FIX-PEG are active.

## Claims

1. A Factor IX-polyethylene glycol (FIX-PEG) conjugate, wherein one or more polyethlylene glycol groups are bound to Factor IX through a PEG containing moiety which bridges two cysteine residues that form a disulfide bond in native Factor IX.

2. The FIX-PEG conjugate of claim 1, according to the formula: 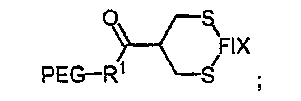
wherein R¹ represents a linking group which can be a direct bond, an alkylene group (preferably a C₁₋₁₀ alkylene group), or an optionally-substituted aryl or heteroaryl group;
wherein the aryl groups include phenyl and naphthyl groups;
wherein suitable heteroaryl groups include pyridine, pyrrole, furan, pyran, imidazole, pyrazole, oxazole, pyridazine, pyrimidine and purine;
wherein linkage to the polymer may be by way of a hydrolytically labile bond, or by a non-labile bond.

3. The FIX-PEG conjugate of claim 2 according to the formula:

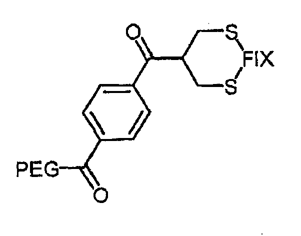

4. The FIX-PEG conjugate of any one of claims 1 to 3, wherein the one or more PEG-containing moiety has a molecular weight of about 10 kDa.

5. The FIX-PEG conjugate of any one of claims 1 to 4, having one PEG-containing moiety.

6. The FIX-PEG conjugate of any one of claims 1 to 4, having two PEG-containing moieties.

7. A composition comprising the conjugate of any of claims 1 to 6, wherein the composition also comprises a pharmaceutically acceptable diluent, adjuvant or carrier.

8. The composition of claim 7, wherein the composition has been formulated for parenteral administration.

9. The composition of claim 8, which is suitable for intradermal, subcutaneous, and intramuscular injections, and intravenous or intraosseous infusions.

10. A composition comprising the conjugate of any of claims 1 to 6, which takes the form of a solution, suspension or emulsion.

11. The conjugate of any of claims 1 to 6, wherein the FIX-PEG conjugate has a longer half-life as compared to unmodified FIX.

12. The conjugate of any of claims 1 to 6, wherein the FIX-PEG conjugate has a higher AUC as compared to unmodified FIX.

13. The conjugate of any of claims 1 to 6, wherein the FIX-PEG conjugate has a higher bioavailability as compared to unmodified FIX.

14. A pharmaceutical composition comprising the FIX-PEG conjugate of any of claims 1 to 6 and a pharmaceutically acceptable diluent, adjuvant or carrier for use in treating hemophilia B.

15. A pharmaceutical composition comprising the FIX-PEG conjugate of any of claims 1 to 6 and a pharmaceutically acceptable diluent, adjuvant or carrier, wherein the composition is substantially free of Factor IXa, for use in reducing the risk of hemarthrosis, hemorrhage, gastrointestinal bleeding and menorrhagia in mammals with hemophilia B.

16. The composition of claim 15, wherein the mean thrombus score of the composition is less than 1 after three independent experiments.

17. The composition of claim 15, wherein the mean thrombus score of the composition is less than 0.5 after three independent experiments.

18. The composition of claim 15, wherein the mean thrombus score of the composition is 0 after three independent experiments.

19. The composition of claim 14, wherein the composition is administered subcutaneously.

20. The composition of claim 14, wherein the composition is administered intravenously.

21. The composition of claim 14, wherein the composition leads to a reduced incidence of thrombosis compared to compositions of recombinant Factor IX which comprise a measurable quantity of Factor IXa.

22. The composition of claim 14, wherein the composition is administered once a day.

23. The composition of claim 14, wherein the composition is administered once every two days.

24. The composition of claim 14, wherein the composition is administered at a dose from 1 IU to 10,000 IU.

25. The composition of claim 14, wherein the composition is administered at a dose from 200 IU to 2500 IU.

26. The composition of claim 14, wherein the composition is administered at a dose of about 250 IU, about 500 IU, about 1000 IU or about 2000 IU.

27. The composition of claim 14, wherein the composition is administered at a dose such that the concentration of circulating Factor IX activity is 1 IU/dL to 150 IU/dL.

28. The composition of claim 14, wherein the composition is administered at a dose such that the concentration of circulating Factor IX activity is 10 IU/dL to 120 IU/dL.

29. The composition of claim 14, wherein the composition is administered at a dose such that the concentration of circulating Factor IX activity is 20 IU/dL to 100 IU/dL.

## Patentansprüche

1. Faktor-IX-Polyethylenglycol-Konjugat (FIX-PEG-Konjugat), wobei eine oder mehrere Polyethylenglycolgruppen durch einen PEG enthaltenden Molekülteil, der zwei Cysteinreste verbrückt, die in nativem Faktor IX eine Disulfidbindung bilden, an Faktor IX gebunden sind.

2. FIX-PEG-Konjugat nach Anspruch 1 gemäß der Formel: 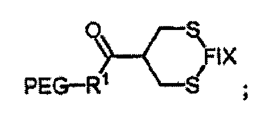
wobei R¹ eine Verknüpfungsgruppe darstellt, die eine direkte Bindung, eine Alkylengruppe (vorzugsweise eine C₁₋₁₀-Alkylengruppe) oder eine gegebenenfalls substituierte Aryl- oder Heteroarylgruppe sein kann;
wobei die Arylgruppen u.a. Phenyl- und Naphthylgruppen sind;
wobei geeignete Heteroarylgruppen u.a. Pyridin, Pyrrol, Furan, Pyran, Imidazol, Pyrazol, Oxazol, Pyridazin, Pyrimidin und Purin sind;
wobei die Verknüpfung mit dem Polymer mittels einer hydrolytisch labilen Bindung oder einer nichtlabilen Bindung sein kann.

3. FIX-PEG-Konjugat nach Anspruch 2 gemäß der Formel:

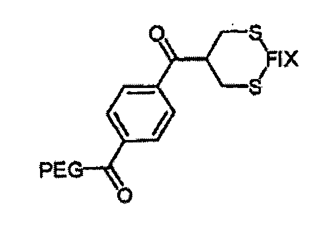

4. FIX-PEG-Konjugat nach einem von Anspruch 1 bis 3, wobei der ein oder mehrere PEG enthaltende Molekülteil ein Molekulargewicht von etwa 10 kDa aufweist.

5. FIX-PEG-Konjugat nach einem von Anspruch 1 bis 4, das einen PEG enthaltenden Molekülteil aufweist.

6. FIX-PEG-Konjugat nach einem von Anspruch 1 bis 4, das zwei PEG enthaltende Molekülteile aufweist.

7. Zusammensetzung, umfassend das Konjugat nach einem von Anspruch 1 bis 6, wobei die Zusammensetzung auch ein pharmazeutisch akzeptables Verdünnungsmittel, Adjuvans oder Trägerstoff umfasst.

8. Zusammensetzung nach Anspruch 7, wobei die Zusammensetzung zur parenteralen Verabreichung formuliert wurde.

9. Zusammensetzung nach Anspruch 8, die für intradermale, subkutane und intramuskuläre Injektionen und intravenöse oder intraossäre Infusionen geeignet ist.

10. Zusammensetzung, umfassend das Konjugat nach einem von Anspruch 1 bis 6, die die Form einer Lösung, Suspension oder Emulsion annimmt.

11. Konjugat nach einem von Anspruch 1 bis 6, wobei das FIX-PEG-Konjugat im Vergleich zu unmodifiziertem FIX eine längere Halbwertszeit aufweist.

12. Konjugat nach einem von Anspruch 1 bis 6, wobei das FIX-PEG-Konjugat im Vergleich zu unmodifiziertem FIX eine größere AUC aufweist.

13. Konjugat nach einem von Anspruch 1 bis 6, wobei das FIX-PEG-Konjugat im Vergleich zu unmodifiziertem FIX eine höhere Bioverfügbarkeit aufweist.

14. Pharmazeutische Zusammensetzung, umfassend das FIX-PEG-Konjugat nach einem von Anspruch 1 bis 6 und ein pharmazeutisch akzeptables Verdünnungsmittel, Adjuvans oder Trägerstoff, zur Benutzung zum Behandeln von Hämophilie B.

15. Pharmazeutische Zusammensetzung, umfassend das FIX-PEG-Konjugat nach einem von Anspruch 1 bis 6 und ein pharmazeutisch akzeptables Verdünnungsmittel, Adjuvans oder Trägerstoff, wobei die Zusammensetzung im Wesentlichen frei von Faktor IXa ist, zur Anwendung im Verringern des Risikos der Hämarthrose, Hämorrhagie, gastrointestinale Blutung und Menorrhagie bei Säugern mit Hämophilie B.

16. Zusammensetzung nach Anspruch 15, wobei der mittlere Thrombus-Score der Zusammensetzung nach drei unabhängigen Versuchen kleiner als 1 ist.

17. Zusammensetzung nach Anspruch 15, wobei der mittlere Thrombus-Score der Zusammensetzung nach drei unabhängigen Versuchen kleiner als 0,5 ist.

18. Zusammensetzung nach Anspruch 15, wobei der mittlere Thrombus-Score der Zusammensetzung nach drei unabhängigen Versuchen 0 ist.

19. Zusammensetzung nach Anspruch 14, wobei die Zusammensetzung subkutan verabreicht wird.

20. Zusammensetzung nach Anspruch 14, wobei die Zusammensetzung intravenös verabreicht wird.

21. Zusammensetzung nach Anspruch 14, wobei die Zusammensetzung im Vergleich zu Zusammensetzungen von rekombinantem Faktor IX, die eine messbare Menge an Faktor IXa umfassen, zu einer verringerten Inzidenz von Thrombose führt.

22. Zusammensetzung nach Anspruch 14, wobei die Zusammensetzung einmal täglich verabreicht wird.

23. Zusammensetzung nach Anspruch 14, wobei die Zusammensetzung alle zwei Tage verabreicht wird.

24. Zusammensetzung nach Anspruch 14, wobei die Zusammensetzung in einer Dosis von 1 I.E. bis 10.000 I.E. verabreicht wird.

25. Zusammensetzung nach Anspruch 14, wobei die Zusammensetzung in einer Dosis von 200 I.E. bis 2.500 I.E. verabreicht wird.

26. Zusammensetzung nach Anspruch 14, wobei die Zusammensetzung in einer Dosis von etwa 250 I.E., etwa 500 I.E., etwa 1.000 I.E. oder etwa 2.000 I.E. verabreicht wird.

27. Zusammensetzung nach Anspruch 14, wobei die Zusammensetzung in solch einer Dosis verabreicht wird, dass die Konzentration von zirkulierender Faktor-IX-Aktivität 1 I.E./dl bis 150 I.E./dl beträgt.

28. Zusammensetzung nach Anspruch 14, wobei die Zusammensetzung in solch einer Dosis verabreicht wird, dass die Konzentration von zirkulierender Faktor-IX-Aktivität 10 I.E./dl bis 120 I.E./dl beträgt.

29. Zusammensetzung nach Anspruch 14, wobei die Zusammensetzung in solch einer Dosis verabreicht wird, dass die Konzentration von zirkulierender Faktor-IX-Aktivität 20 I.E./dl bis 100 I.E./dl beträgt.

## Revendications

1. Conjugué Facteur IX-polyéthylène glycol (FIX-PEG), dans lequel un ou plusieurs groupes polyéthylène glycol sont liés au Facteur IX par un fragment contenant du PEG qui ponte deux résidus de cystéine qui forment une liaison disulfure dans le Facteur DC natif.

2. Conjugué FIX-PEG selon la revendication 1, selon la formulé : 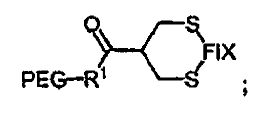
dans lequel R¹ représente un groupe de liaison qui peut être une liaison directe, un groupe alkylène (de préférence, un groupe alkylène en C_{1 à 10}), ou un groupe aryle ou hétéroaryle éventuellement substitué ;
dans lequel les groupes aryle comprennent les groupes phényle et naphtyle ;
dans lequel les groupes hétéroaryle appropriés comprennent pyridine, pyrrole, furane, pyrane, imidazole, pyrazole, oxazole, pyridazine, pyrimidine et purine ;
dans lequel la liaison au polymère peut être au moyen d'une liaison labile par hydrolyse ou par une liaison non labile.

3. Conjugué FIX-PEG selon la revendication 2, selon la formule :

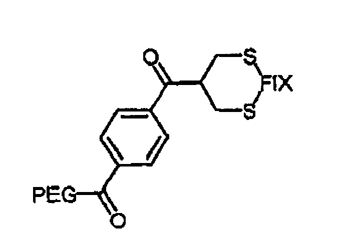

4. Conjugué FIX-PEG selon l'une quelconque des revendications 1 à 3, dans lequel le ou les fragments contenant du PEG ont une masse moléculaire d'environ 10 kDa.

5. Conjugué FIX-PEG selon l'une quelconque des revendications 1 à 4, ayant un fragment contenant du PEG.

6. Conjugué FIX-PEG selon l'une quelconque des revendications 1 à 4, ayant deux fragments contenant du PEG.

7. Composition comprenant le conjugué selon l'une quelconque des revendications 1 à 6, où la composition comprend également un diluant, adjuvant ou véhicule acceptable sur le plan pharmaceutique.

8. Composition selon la revendication 7, où la composition a été formulée pour administration par voie parentérale.

9. Composition selon la revendication 8, qui est appropriée pour des injections intradermiques, sous-cutanées, et intramusculaires, et des perfusions intraveineuses ou intra-osseuses.

10. Composition comprenant le conjugué selon l'une quelconque des revendications 1 à 6, qui prend la forme d'une solution, suspension ou émulsion.

11. Conjugué selon l'une quelconque des revendications 1 à 6, dans lequel le conjugué FIX-PEG a une demi-vie plus longue par comparaison avec le FIX non modifié.

12. Conjugué selon l'une quelconque des revendications 1 à 6, dans lequel le conjugué FIX-PEG a une aire sous la courbe plus élevée par comparaison avec le FIX non modifié.

13. Conjugué selon l'une quelconque des revendications 1 à 6, dans lequel le conjugué FIX-PEG a une biodisponibilité plus élevée par comparaison avec le FIX non modifié.

14. Composition pharmaceutique comprenant le conjugué FIX-PEG selon l'une quelconque des revendications 1 à 6 et un diluant, adjuvant ou véhicule acceptable sur le plan pharmaceutique, destinée à être utilisée pour le traitement de l'hémophilie B.

15. Composition pharmaceutique comprenant le conjugué FIX-PEG selon l'une quelconque des revendications 1 à 6 et un diluant, adjuvant ou véhicule acceptable sur le plan pharmaceutique, où la composition est essentiellement dépourvue de Facteur IXa, destinée à être utilisée pour réduire le risque d'hémarthrose, d'hémorragie, de saignement gastro-intestinal et de ménorragie chez des mammifères souffrant d'hémophilie B.

16. Composition selon la revendication 15, dans laquelle la note moyenne de thrombus de la composition est inférieure à 1 après trois expériences indépendantes.

17. Composition selon la revendication 15, dans laquelle la note moyenne de thrombus de la composition est inférieure à 0,5 après trois expériences indépendantes.

18. Composition selon la revendication 15, dans laquelle la note moyenne de thrombus de la composition est 0 après trois expériences indépendantes.

19. Composition selon la revendication 14, où la composition est administrée par voie sous-cutanée.

20. Composition selon la revendication 14, où la composition est administrée par voie intraveineuse.

21. Composition selon la revendication 14, où la composition entraîne une incidence réduite de thrombose par comparaison avec des compositions de Facteur IX recombiné qui comprennent une quantité mesurable de Facteur IXa.

22. Composition selon la revendication 14, où la composition est administrée une fois par jour.

23. Composition selon la revendication 14, où la composition est administrée une fois tous les deux jours.

24. Composition selon la revendication 14, où la composition est administrée à une dose de 1 UI à 10 000 UI.

25. Composition selon la revendication 14, où la composition est administrée à une dose de 200 UI à 2500 UI.

26. Composition selon la revendication 14, où la composition est administrée à une dose d'environ 250 UI, environ 500 UI, environ 1000 UI ou environ 2000 UI.

27. Composition selon la revendication 14, où la composition est administrée à une dose telle que la concentration d'activité de Facteur IX en circulation va de 1 UI/dL à 150 UI/dL.

28. Composition selon la revendication 14, où la composition est administrée à une dose telle que la concentration d'activité de Facteur IX en circulation va de 10 UI/dL à 120 UI/dL.

29. Composition selon la revendication 14, où la composition est administrée à une dose telle que la concentration d'activité de Facteur IX en circulation va de 20 UI/dL à 100 UI/dL.
